Europäisches Patentamt

European Patent Office

Office européen des brevets

(11) Veröffentlichungsnummer: **0 141 263**
**A1**

(12) ## EUROPÄISCHE PATENTANMELDUNG

(21) Anmeldenummer: **84111587.6**

(22) Anmeldetag: **28.09.84**

(51) Int. Cl.⁴: **C 12 Q 1/56**
G 01 N 33/543, G 01 N 33/545
C 12 Q 1/38

(30) Priorität: **06.10.83 DE 3336361**

(43) Veröffentlichungstag der Anmeldung:
**15.05.85 Patentblatt 85/20**

(84) Benannte Vertragsstaaten:
**AT BE CH DE FR GB IT LI LU NL SE**

(71) Anmelder: **BEHRINGWERKE Aktiengesellschaft**
**Postfach 1140**
**D-3550 Marburg 1(DE)**

(72) Erfinder: **Pâques, Eric, Dr.**
**Schmiedeacker 18**
**D-3550 Marburg-Haddamshausen(DE)**

(72) Erfinder: **Stöhr, Hans-Arnold**
**Schulstrasse 66**
**D-3552 Wetter(DE)**

(74) Vertreter: **Meyer-Dulheuer, Karl-Hermann, Dr. et al,**
**HOECHST Aktiengesellschaft Zentrale Patentabteilung**
**Postfach 80 03 20**
**D-6230 Frankfurt/Main 80(DE)**

(54) Verfahren zur Herstellung von trägergebundenem Fibrin sowie seine Verwendung in einem Verfahren zur Bestimmung von Proteinen.

(57) Verfahren zur Herstellung von trägergebundenem Fibrin sowie seine Verwendung in einem Verfahren zur Bestimmung von Proteinen.

Es wird ein Verfahren zur Herstellung von trägergebundenem Fibrin durch Beschichten einer Oberfläche eines Trägers mit, gegebenenfalls markiertem, Fibrin, beschrieben. Zu diesem Zweck wird ein Träger mit einer Lösung von, gegebenenfalls markierten, Fibrinogen und einem Gerinnungsmittel oder einer Lösung von, gegebenenfalls markierten, Fibrin- oder Fibrinogenspaltprodukten und gegebenenfalls einem Markierungsmittel behandelt. Ein auf diese Weise beschichteter Träger kann zum Nachweis und zur Bestimmung von Proteinen und besonders auch von proteolytischen Enzymen verwendet werden.

EP 0 141 263 A1

**0141263**

BEHRINGWERKE AKTIENGESELLSCHAFT    83/B 017
                                    Dr. Ha/gä

Verfahren zur Herstellung von trägergebundenem Fibrin
sowie seine Verwendung in einem Verfahren zur Bestimmung
von Proteinen

Die Erfindung betrifft ein Verfahren zur Herstellung von
Oberflächen, die mit Fibrin-oder Fibrinogenspaltprodukten beschichtet sind.
Sie betrifft weiterhin ein Verfahren zum Nachweis und
zur Bestimmung von Proteinen, die eine Affinität zu
Fibrin haben, mittels solcher beschichteter Oberflächen.
Auch proteolytische Enzyme können auf diese Weise
bestimmt werden.

Die Beschichtung von Oberflächen mit bestimmten Antigenen oder Antikörpern für die immunologische Bestimmung
von Antikörpern oder Antigenen ist bekannt. Mit Fibrin
sind Oberflächen jedoch anscheinend noch nicht beschichtet worden.

Fibrin entsteht durch proteolytische Abspaltung von
Fibrinopeptid A und B aus Fibrinogen. Die entstehenden
löslichen Fibrin-Monomer-Moleküle lagern sich zusammen
und bilden Fibrin-Polymere, die nach Einwirkung von F
XIII als kovalent vernetzte Polymere vorliegen. Eine
Reihe von Proteinen wie Plasminogen, Plasmin,
Plasminogen-Aktivatoren, Fibronectin, $\alpha_2$-Antiplasmin,
$\alpha_2$-Antiplasmin-Plasmin-Komplex oder Plasminogen-
Streptokinase weisen eine hohe Affinität zu Fibrin auf.
Außerdem wirkt Fibrin als Substrat für Enzyme wie Plasmin und als Effektor für Plasminogen-Aktivatoren wie
Gewebe-Plasminogen-Aktivator oder Ein-Ketten-Urokinase.

Die im Stand der Technik bekannten Methoden ermöglichen
nicht die gleichzeitige und schnelle Bestimmung der Affinität sowie der Aktivität solcher mit Fibrin in Wechselwirkung tretenden Proteine.

Daher stellte sich die Aufgabe, eine solche Methode zu entwickeln.

Überraschenderweise wurde gefunden, daß fibrin-beschichtete Oberflächen für die gleichzeitige, schnelle und empfindliche Affinitäts- sowie Aktivitäts-Bestimmung von Proteinen, die mit Fibrin in Wechselwirkung treten können, geeignet sind.

Gegenstand der Erfindung ist deshalb ein Verfahren für die Beschichtung eines Trägers mit Fibrin- oder Fibrinogenspaltprodukten.

Gegenstand der Erfindung ist auch ein Verfahren zur Herstellung eines Trägers, der mit markierten Fibrin- oder Fibrinogenspaltprodukten beschichtet ist.

Zur Beschichtung kann, gegebenenfalls markiertes, Fibrinogen dienen, das dann zur Gerinnung gebracht wird.

Das Markierungsmittel kann an das Beschichtungsmittel adsorbiert, darin eingeschlossen oder kovalent oder nicht kovalent daran gebunden sein.

Bevorzugt sind Marker, die an das Beschichtungsmittel adsorbiert oder kovalent oder nichtkovalent daran gebunden sind sowie Marker, die im Fibrinnetz eingeschlossen sind. Radionuklide, Enzyme, Farbstoffe, Enzym-Substrate und Inhibitoren sind als Marker besonders bevorzugt.

Als zu beschichtende Oberflächen von Trägern kommen in Frage beispielsweise Polystyrol, Polyurethan, Silikon, Cellulose-Acetat, PVC, Latex, Diatomeen-Erde, Glas sowie auch physikalisch oder chemisch aktivierte wasserunlösliche Oberflächen wie $\gamma$-bestrahltes Polystyrol, Aminopropylglas.

Ein bevorzugtes Herstellungsverfahren besteht darin, daß eine Oberfläche eines Trägers, beispielsweise Polystyrol-Röhrchen, mit einer Fibrinogenlösung, die 50 bis 0,0001 mg/ml Protein enthält, inkubiert und gegebenenfalls gewaschen werden, das vorhandene Fibrinogen zur Gerinnung gebracht wird und die Oberflächen gewaschen und gegebenenfalls getrocknet werden. Gegebenenfalls werden die an den Oberflächen vorhandenen Fibrinmoleküle mit einem Marker behandelt.

Ein anderes Herstellungsverfahren besteht darin, daß ein Träger, beispielsweise Polystyrol-Röhrchen, mit einer Mischung von markiertem Fibrinogen und Fibrinogen in einem Verhältnis von 0 bis 1, mit einer Lösung mit 50 bis 0.0001 mg/ml Protein in Berührung gebracht, inkubiert und gegebenenfalls gewaschen werden, das Fibrinogen in Gegenwart oder Abwesenheit von F XIII zur Gerinnung gebracht wird und die Oberflächen gewaschen und gegebenenfalls getrocknet werden.

Ein weiteres Herstellungsverfahren besteht darin, daß ein Träger, beispielsweise Polystyrol-Röhrchen, mit einer Mischung von Fibrinogen und einem Marker wie einem Enzym, Enzym-Substrat, Inhibitor oder Farbstoff mit einer Lösung enthaltend 50 bis 5 mg/ml Fibrinogen in Berührung gebracht und inkubiert werden, das vorhandene Fibrinogen in Gegenwart oder Abwesenheit von F XIII zur Gerinnung gebracht wird und die Oberflächen gewaschen und gegebenenfalls getrocknet werden.

Ein weiteres Herstellungsverfahren besteht darin, daß ein Träger, beispielsweise Polystyrol-Röhrchen, mit einer Lösung von Fibrin- oder Fibrinogenspaltprodukten oder gegebenenfalls mit einer Mischung von Fibrinspaltprodukten und markierten Fibrin- oder Fibrinogenspaltprodukten, in einem Verhältnis von 0 bis 1 mit einer Lösung enthaltend 0,1 bis 50 µg/ml Protein, in Kontakt gebracht, inkubiert, gewaschen und gegebenenfalls getrocknet werden.

In einer bevorzugten Ausführungsform verfährt man so, daß 0,04 ml einer 0,5 bis 5 g Fibrinogen auf 100 ml enthaltende Lösung vorzugsweise 2 g in 100 ml enthaltende Lösung, in einem Röhrchen mit, vorzugsweise 0,2 NIH Thrombin, vorzugsweise bei 20° bis 37°C, vorzugsweise über Nacht bei 37°C, zur Gerinnung gebracht und getrocknet werden und das Röhrchen vorzugsweise gewaschen und anschließend getrocknet wird.

In einer besonders bevorzugten Ausführungsform verfährt man so, daß eine 0,5 bis 5 g Fibrinogen auf 100 ml enthaltende Lösung, vorzugsweise eine 2 g/100 ml enthaltende Lösung, die einen Farbstoff, beispielsweise 0,02 g/ml Blue-Dextran, enthält, im Röhrchen mit Thrombin vorzugsweise bei 20 bis 37°C, zur Gerinnung gebracht, vorzugsweise über Nacht bei 37°C getrocknet, vorzugsweise mit Wasser gewaschen und anschließend getrocknet wird.

In einer anderen besonders bevorzugten Herstellungsmethode verfährt man so, daß die mit Fibrin, Fibrinogen- oder Fibrinspaltprodukten beschichteten Oberflächen anschließend mit perjodat-behandelter Peroxidase umgesetzt, gewaschen und gegebenenfalls getrocknet werden. Gegebenenfalls können die beschichteten Oberflächen mit Peroxidase markierten Fibrinogen-Antikörpern behandelt werden.

Gegenstand der Erfindung ist weiterhin ein Verfahren zum Nachweis und zur Bestimmung von Proteinen, die eine Affinität zu Fibrin oder Fibrinspaltprodukten haben, dadurch gekennzeichnet, daß man einen wasserunlöslichen Träger mit einer Lösung eines Fibrin- oder Fibrinogenspaltproduktes oder von Fibrinogen und im Falle von Fi- brinogen außerdem mit einem Gerinnungsmittel in Kontakt bringt und inkubiert, wodurch ein beschichteter Träger erhalten wird, gegebenenfalls wäscht, den beschichteten Träger mit einer Lösung des zu bestimmenden Proteins zusammenbringt und das Protein bestimmt.

Bevorzugt ist die Bestimmung oder der Nachweis von Proteinen, die eine Affinität zu Fibrin haben, aber auch von proteolytischen Enzymen, die Fibrin abbauen können. Durch die Einwirkung dieser Proteine auf die Beschichtung kann unter bekannten Reaktionsbedingungen die Freisetzung von Fibrinspaltprodukten oder markierten Fibrinspaltprodukten oder der eingeschlossenen Marker verfolgt werden.

Die an die beschichtete Oberfläche gebundenen Proteine können immunologisch, beispielsweise mit peroxidasemarkierten Antikörpern, nachgewiesen oder bestimmt werden, wobei die enzymatische Aktivität der Peroxidase gemessen wird.

Besonders bevorzugt ist die Bestimmung von Plasminogen, Plasmin, Plasmin-Inhibitoren-Komplex, Plasminogen- Aktivatoren, Fibronectin oder $\alpha_2$-Antiplasmin-Inhibitor.

In einer bevorzugten Anwendungsform verfährt man so, daß beispielsweise 0,1 ml Plasminogen-Aktivator-haltige Lösung, vorzugsweise in Fibrin-beschichteten Röhrchen, beispielsweise 0,5 bis 5 Stunden bei 37°C in Gegenwart von 0,1 ml einer Plasminogenlösung enthaltend vorzugsweise 1 CTA/ml beispielsweise in einem 50 mmol/l Phosphatpuffer, pH 7,5, der 1 g/100 ml Albumin enthält, inkubiert wird. Der Plasminogen-Aktivator bindet sich an Fibrin, das dessen spezifische Aktivität verstärkt. Unter Einwirkung dieses Enzyms wird Plasminogen in Plasmin umgewandelt. Das entstandene Plasmin spaltet Fibrin in Fibrinspaltprodukte. Die freigesetzten Peptide werden mit einem für Fibrinspaltprodukte empfindlichen Test, wie beispielsweise dem Staphylokokken-Clumping-Test, nachgewiesen. In einer anderen bevorzugten Anwendungsform verfährt man in einer ähnlichen Weise für die Bestimmung von Plasminogen, Plasmin oder $\alpha_2$-Antiplasmin.

Gegenstand der Erfindung ist deshalb weiterhin die Anwendung fibrinbeschichteter Träger zum Nachweis von Proteinen, die Affinität zu Fibrin aufweisen sowie die Aktivitätsbestimmung solcher Proteine.

Besonders bevorzugt ist die Anwendung fibrinbeschichteter Träger für den Nachweis von fibrinbindenden Proteinen oder Proteinasen, wie sie in Blut, Plasma, Serum oder Gewebeflüssigkeiten vorhanden sind.

Besonders bevorzugt ist die Verwendung solcher beschichteter Träger zur Diskriminierung zwischen fibrinbindenden und -nichtbindenden Plasminogen-Aktivatoren.

In einer bevorzugten Ausführungsform verfährt man so, daß eine fibrinbeschichtete Oberfläche mit der Plasminogen-Aktivator-haltigen Lösung, gegebenenfalls unter Zugabe eines Plasmin-Inhibitors wie Aprotinin, beispielsweise 1 bis 60 Minuten, vorzugsweise 5 bis 15 Minuten, bei 20 bis 37°C inkubiert wird. Danach wird die fibrinhaltige Oberfläche gewaschen. Das am Fibrin gebundene Protein wird wie oben beschrieben bestimmt.

Weiterhin können fibrinbeschichtete Oberflächen zur Entfernung von fibrinbindenden Substanzen verwendet werden.

Die Erfindung soll durch nachstehende Beispiele erläutert werden.

Beispiel 1:

Rinder-Fibrinogen wurde in destilliertem Wasser aufgelöst. 0,04 ml einer plasminogenfreien 2 g/100 ml enthaltenden Rinderfibrinogen-Lösung wurde in ein Polystyrol-Röhrchen gegeben. Danach wurden 0,02 ml einer Thrombinlösung (10 NIH/ml) zugegeben und die Lösung durch Schütteln 30 bis 100 Sekunden homogenisiert. Nach Gerinnung wurden die fibrinhaltigen Röhrchen über Nacht im Brutraum bei 37°C getrocknet. Die erhaltenen fibrinbeschichteten Röhrchen wurden so oft mit Wasser gewaschen, bis kein Fibrin oder Fibrinspaltprodukte in der Waschlösung nachgewiesen werden konnten. Die gewaschenen Röhrchen wurden anschließend über Nacht bei 37°C im Brutraum getrocknet.

Beispiel 2:

Wie Beispiel 1, wobei die Fibrinogen-Lösung 20 mg/ml Blue-Dextran 2000 enthielt. Die Röhrchen wurden bis zum vollständigen Entfernen überschüssigen Farbstoffes gewaschen.

## Beispiel 3:

Wie Beispiel 1, wobei die mit Fibrin beschichteten
Röhrchen mit 0,05 ml einer mit Peroxidase markierten
Antifibrinogen-Immunglobulin-Lösung 2 Stunden unter
Lichtausschluß inkubiert, danach gewaschen und
getrocknet wurden.

## Beispiel 4:

Die erhaltenen fibrinbeschichteten Röhrchen wurden für
die Bestimmung von Gewebe-Plasminogen-Aktivator angewendet. Gewebe-Plasminogen-Aktivator-haltige Lösung
wurde in einer geometrischen Reihe mit einem 50 mmol/l
Phosphatpuffer, der 1 g/100 ml Humanalbumin enthielt pH
7,5, verdünnt.

Der Testansatz bestand aus 0,1 ml Aktivator-haltige Verdünnung und 0,1 ml einer Plasminogen-Lösung, die 10
CTA/ml enthielt. Diese Proben wurden 1 bis 5 Stunden bei
37°C in Fibrin-beschichteten Röhrchen inkubiert. In
regelmäßigen Zeitabständen wurden Proben von 0,03 ml
entnommen und mit 0,03 ml einer Staphylokokken- Suspension gemischt. Die Agglutination wurde beobachtet. Drei
Kontrollen, die aus 0,2 ml Puffer, 0,1 ml Puffer und 0,1
ml Plasminogen und 0,1 ml Puffer und 0,1 ml Aktivator
bestanden, wurden eingesetzt. Nach 4 Stunden Inkubation
in Fibrin-Röhrchen blieben alle Kontrollen negativ, das
heißt, bis mindestens 10 Minuten nach Zugabe der
Staphylokokken-Suspension konnte keine Agglutination
beobachtet werden. Die Reaktion wurde als positiv
anerkannt, wenn innerhalb von zwei Minuten nach Zugabe
von Staphylokokken eine Agglutination auftrat.

Der folgenden Tabelle kann die Empfindlichkeit der er-

findungsgemäßen Methode entnommen werden:

| Zeit (h) | TPA * (Einheiten) | | | | | | Kontrollen | | |
|---|---|---|---|---|---|---|---|---|---|
| | 0,05 | 0,01 | 0,005 | 0,001 | 0,0005 | 0,0001 | TPA 0,05E | Plasmi- nogen | Puffer |
| 1 | + | - | - | - | - | - | - | - | - |
| 2 | + | + | + | + | - | - | - | - | - |
| 3 | + | + | + | + | + | - | - | - | - |
| 4 | + | + | + | + | + | + | - | - | - |

* Tissue Plasminogen Activator

+ bedeutet Agglutination

- bedeutet keine Agglutination

Beispiel 5:

Die Bestimmung wurde wie in Beispiel 4 durchgeführt. Die Methode wurde auf ihre Fähigkeit, fibrinbindende und nichtbindende Proteine zu unterscheiden, getestet. Urokinase (aus Urin gewonnen) (1) und Gewebe-Plasminogen-Aktivator (2) wurden wie in Beispiel 4 getestet.

Zusätzlich wurden eine Urokinase-Probe (3) und ein Gewebe-Plasminogen-Aktivator (4) 10 min. bei 37°C in Fibrin-Röhrchen inkubiert, danach 3 mal mit Puffer gewaschen und anschließend mit 0,1 ml Puffer und 0,1 ml Plasminogen (10 CTA/ml) wie in Beispiel 4 inkubiert und getestet.

Ergebnisse:

| Probe | Staphylokokken-Agglutination |
|-------|------------------------------|
| 1 | positiv |
| 2 | positiv |
| 3 | negativ |
| 4 | positiv |

Beispiel 6:

Fibrin-Röhrchen wie in Beispiel 2 beschrieben, wurden mit Gewebe-Plasminogen-Aktivator in einer ähnlichen Weise wie in Beispiel 4 inkubiert. Die Reaktion wurde spektrophotometrisch verfolgt.
Eine Zeit-und Aktivator-Dosis-abhängige Zunahme der optischen Dichte wurde beobachtet.

Beispiel 7:

Fibrin-Röhrchen wie in Beispiel 3 beschrieben, wurden
mit Gewebe-Plasminogen-Aktivator wie in Beispiel 4 inku-
biert. Das Ausmaß der Spaltung des Fibrins wurde mittels
der Bestimmung von freigesetzten Peroxidase-haltigen
Fragmenten bestimmt. Die Peroxidase-Bestimmung wurde
nach bekannter Methode durchgeführt.

Ergebnisse:

| Zeit(h) | TPA (Einheiten) | | | | Kontrolle | | |
| --- | --- | --- | --- | --- | --- | --- | --- |
| | 1,0 | 0,1 | 0,01 | 0,001 | Plasminogen | TPA (1E) | Puffer |
| 2 | 0,456 | 0,146 | 0,017 | 0,004 | 0,001 | 0,000 | 0,000 |
| 4 | – | 0,315 | 0,178 | 0,040 | 0,011 | 0,000 | 0,000 |

0141263
HOE 83/B 017

## Patentansprüche

1. Verfahren zur Herstellung trägergebundenen Fibrins durch Beschichten einer Oberfläche eines Trägers mit Fibrin, dadurch gekennzeichnet, daß ein Träger mit einer Fibrinogenlösung und einem Gerinnungsmittel oder einer Lösung von Fibrin- oder Fibrinogenspaltprodukten in Kontakt gebracht, inkubiert, gewaschen und gegebenenfalls getrocknet wird.

2. Verfahren nach Anspruch 1, dadurch gekennzeichnet, daß das Fibrinogen oder die Fibrin- oder Fibrinogenspaltprodukte markiert sind.

3. Verfahren nach Anspruch 1, dadurch gekennzeichnet, daß mit einem Markierungsmittel behandelt wird, nachdem der Träger mit einer Fibrinogenlösung und einem Gerinnungsmittel oder einer Lösung von Fibrin- oder Fibrinogenspaltprodukten in Kontakt gebracht worden war.

4. Verfahren zum Nachweis und zur Bestimmung eines Proteins, das eine Affinität zu Fibrin oder einem Fibrinspaltprodukt hat, dadurch gekennzeichnet, daß ein nach Anspruch 1 beschichteter Träger mit einer Lösung dieses Proteins zusammengebracht und das Protein bestimmt wird.

5. Verfahren nach Anspruch 1, dadurch gekennzeichnet, daß der Träger aus Polystyrol besteht.

6. Verwendung trägergebundenen Fibrins nach Anspruch 1 zum Nachweis und zur Bestimmung eines Proteins.

7. Verwendung von Fibrin, das an Polystyrol adsorbiert
   ist, zum Nachweis und zur Bestimmung eines Proteins.

| Category | Citation of document with indication, where appropriate, of relevant passages | Relevant to claim | CLASSIFICATION OF THE APPLICATION (Int. Cl.4) |
|---|---|---|---|
| | **DOCUMENTS CONSIDERED TO BE RELEVANT** | | EP 84111587.6 |
| X | AT - B - 355 225 (BEHRINGWERKE AKTIENGESELLSCHAFT)<br><br>* Ansprüche 1,6 *<br><br>---- | 1,2,4, 6 | C 12 Q 1/56<br><br>G 01 N 33/543<br><br>G 01 N 33/545<br><br>C 12 Q 1/38 |
| | | | **TECHNICAL FIELDS SEARCHED (Int. Cl.4)** |
| | | | C 12 Q<br><br>G 01 N 33/00 |

The present search report has been drawn up for all claims

| Place of search | Date of completion of the search | Examiner |
|---|---|---|
| VIENNA | 11-01-1985 | SCHNASS |